Europäisches Patentamt

European Patent Office

Office européen des brevets

(19)

(11) Numéro de publication : **0 464 180 B1**

(12) **FASCICULE DE BREVET EUROPEEN**

(45) Date de publication du fascicule du brevet :
26.07.95 Bulletin 95/30

(51) Int. Cl.⁶ : **H04L 5/14,** H04B 5/00,
A61N 1/36

(21) Numéro de dépôt : **91903008.0**

(22) Date de dépôt : **18.01.91**

(86) Numéro de dépôt international :
**PCT/FR91/00026**

(87) Numéro de publication internationale :
**WO 91/11063 25.07.91 Gazette 91/17**

(54) **TRANSMISSION INDUCTIVE BIDIRECTIONNELLE DE DONNEES A STATION ESCLAVE ALIMENTEE PAR LE MAITRE.**

(30) Priorité : **19.01.90 FR 9000606**

(43) Date de publication de la demande :
**08.01.92 Bulletin 92/02**

(45) Mention de la délivrance du brevet :
**26.07.95 Bulletin 95/30**

(84) Etats contractants désignés :
**AT BE CH DE DK ES FR GB GR IT LI LU NL SE**

(56) Documents cités :
**EP-A- 0 160 990**
**GB-A- 2 123 648**
**US-A- 4 741 340**
**IEEE Southeastcon '87, Tampa, US, 5-8 avril 1987, pages 394 - 397, IEEE, New York, US; O. A. Mohammed, "Numerical Determination of the Performance of Magnetically Coupled Coils in Medical Telemetry Systems"**
**PATENT ABSTRACTS OF JAPAN vol. 10, no. 197 (E-418)(2253) 10 juillet 1986, & JP-A-61 39741 (CASIO COMPUTER COMPANY LIMITED) 25 février 1986, voir le document entier**

(73) Titulaire : **BERTIN & CIE**
**Zone Industrielle**
**Boîte postale 3**
**F-78373 Plaisir Cédex (FR)**

(72) Inventeur : **GUERN, Yves**
**Quartier Saute-Lièvre**
**F-13490 Jouques (FR)**
Inventeur : **DUBUJET, Bruno**
**34, domaine de Cabri**
**Le Tholonet**
**F-13100 Aix-en-Provence (FR)**
Inventeur : **AYRAUD, Michel**
**22, avenue Aimé-Martin**
**F-06200 Nice (FR)**

(74) Mandataire : **Colas, Jean-Pierre et al**
**Cabinet de Boisse**
**37, avenue Franklin D. Roosevelt**
**F-75008 Paris (FR)**

## Description

L'invention concerne un dispositif de transmission bidirectionnelle d'informations entre un système maître (émetteur) et un système esclave (récepteur) alimenté électriquement à partir de l'énergie électromagnétique émise par le système maître.

Dans de nombreux domaines de la technique, le besoin existe de disposer d'un tel dispositif dont le système esclave, dépourvu de toute source autonome d'alimentation en énergie électrique, soit capable de recevoir des informations, d'alimenter électriquement des circuits électroniques ou autres charges, et de transmettre des informations au système maître.

Tel est le cas, par exemple dans le domaine de la télémesure, de la télécommande, des dispositifs électroniques actifs implantables dans le corps humain ou animal, etc..., où pour des raisons d'inaccessibilité et/ou d'encombrement du système esclave, de fiabilité et/ou de durée de vie insuffisantes de piles électriques, etc..., il n'est pas toujours possible ou souhaitable d'équiper le système esclave d'une source d'énergie électrique autonome.

Dans la suite de la description, on désigne donc par "système esclave" un système comprenant au moins une antenne et des circuits de réception et qui dépend entièrement du "système maître" pour son alimentation en énergie électrique, énergie qui lui est fournie sous forme électromagnétique.

Il est déjà connu, s'agissant notamment des prothèses actives implantables, d'alimenter électriquement un module récepteur à partir de l'énergie rayonnée par l'antenne d'émission d'un module émetteur.

C'est ainsi que le document GB-A-2 123 648 décrit une prothèse implantable de stimulation biologique comprenant une antenne de réception apte à capter un rayonnement véhiculant à la fois des informations de commande et l'énergie nécessaire à l'alimentation électrique des circuits et charges de la prothèse. L'énergie reçue est stockée dans un condensateur qui, associé à un régulateur de tension, sert à alimenter des circuits de démodulation du signal reçu, de décodage des informations véhiculées par ce signal, de commande et d'alimentation des électrodes de stimulation, etc... La transmission d'informations a lieu uniquement de l'émetteur vers le récepteur, elle est donc unidirectionnelle.

Cependant, il peut s'avérer nécessaire qu'une telle prothèse ou un dispositif électronique implanté similaire soit également capable de transmettre vers l'extérieur des données de mesure sur les caractéristiques électriques des électrodes, sur l'état de fonctionnement de ses circuits électriques, des données fournies par des capteurs, etc...

A cet effet, le document WO 84/01719 décrit un dispositif visant une application similaire, dont le système esclave est équipé d'une antenne émettrice et de circuits d'émission desdites données. Le système esclave de ce dispositif comprend donc à la fois un module récepteur et un module émetteur.

Cependant, au niveau du système esclave, l'énergie non négligeable nécessaire à la transmission de données par émission d'un rayonnement électromagnétique vient s'ajouter à l'énergie que consomment les autres circuits et charges que doit alimenter le module récepteur du système esclave. Or, la puissance instantanée extraite par le système esclave du rayonnement émis par le système maître est relativement faible. En pratique, cette puissance s'avère insuffisante pour alimenter à la fois les circuits de réception, de commande, d'émission et les charges électriques du système esclave.

C'est pourquoi, le document WO 84/01719 prévoit d'équiper le système esclave d'un accumulateur capable de stocker l'énergie captée par l'antenne de réception. Mais, pour des raisons d'encombrement, de poids, de sécurité, de fiabilité ou autres, la présence d'un accumulateur électrique dans le système esclave est souvent inacceptable.

Il est par ailleurs connu des documents US-A-4 741 340 et IEEE Southeastcon 87, Tampa, US, 5-8 Avril 1987, pages 394-397, IEEE, New York, d'effectuer une transmission bidirectionnelle d'informations entre un système maître et un système esclave comportant respectivement une antenne émettrice et une antenne réceptrice accordées sur une même fréquence $f$, en modulant l'impédance du système esclave, pendant la réception d'un signal de données émis par le système maître, en fonction des informations à transmettre au système maître. Cette modulation de l'impédance du système esclave se traduit par des variations de caractéristiques électriques du système maître du fait que les antennes émettrice et réceptrice sont couplées. La détection de ces variations permet de reconstituer les informations transmises du système esclave au système maître.

Néanmoins, le dispositif selon le document US-A-4 741 340 et les dispositifs similaires connus ne permettent pas d'alimenter le système esclave en énergie électrique à partir du système maître, et d'assurer de manière sûre la transmission d'un débit élevé d'informations.

L'invention vise à fournir un dispositif permettant tout à la fois la transmission bidirectionnelle d'informations à fréquence élevée entre un système maître et un système esclave et l'alimentation électrique des circuits et/ou charges du système esclave à partir de l'énergie électromagnétique émise par le système maître, et ceci avec une antenne unique tant du côté système esclave que du côté système maître.

A cet effet, l'invention a pour objet un dispositif de transmission bidirectionnelle d'informations entre un système maître et un système esclave comportant respectivement une antenne émettrice unique et une antenne réceptrice unique accordées sur une même fréquence,

- ledit système maître comprenant :

  * des moyens pour transmettre des premières informations au système esclave par modulation d'un signal électromagnétique émis à ladite fréquence,

  * des moyens pour détecter, pendant ladite émission dudit signal électromagnétique modulé, des variations de caractéristiques électriques induites par une modulation de l'impédance du système esclave fonction de secondes informations à transmettre du système esclave au système maître, et

  * des moyens pour reconstituer lesdites secondes informations en fonction desdites variations de caractéristiques électriques détectées,

- ledit système esclave comprenant un circuit résonant de réception accordé sur ladite fréquence et comprenant ladite antenne réceptrice,

      caractérisé en ce que ledit système esclave comprend en outre :

  * un circuit redresseur connecté aux bornes dudit circuit résonant de réception,

  * un circuit d'alimentation générateur de tension continue connecté audit circuit redresseur pour alimenter des circuits et/ou charges dudit système esclave, et

  * un circuit de commutation connecté en série entre ledit circuit redresseur et ledit circuit d'alimentation pour faire varier la partie réelle de l'impédance dudit système esclave entre une valeur élevée et une valeur faible suivant que ledit commutateur est ouvert ou fermé sous la commande d'un signal représentatif desdites secondes informations à transmettre au système maître.

Ainsi, le dispositif suivant l'invention utilise le couplage électromagnétique entre antenne émettrice unique et antenne réceptrice unique pour transmettre de manière bidirectionnelle des informations du système esclave au système maître, et ceci sans rayonnement électromagnétique à partir du système esclave, de sorte que l'essentiel de l'énergie électromagnétique reçue par le système esclave est utilisé pour alimenter électriquement les circuits et/ou charges de celui-ci.

On notera que, tant du côté système maître que du côté système esclave, une seule antenne suffit pour transmettre des informations dans les deux sens, ainsi que la puissance électrique nécessaire au système esclave. Il en résulte un gain de place particulièrement appréciable côté système esclave.

L'énergie nécessaire à la transmission d'informations du système esclave vers le système maître est essentiellement limitée, au niveau du système esclave, à la consommation du commutateur électronique, par exemple un transistor MOS, qui module l'impédance du système esclave. Cette énergie est donc très faible, et une fraction appréciable de l'énergie captée par le système esclave demeure disponible pour alimenter ses autres circuits et charges, notamment ceux élaborant les données à transmettre au système maître.

Le dispositif suivant l'invention ouvre la voie à de nombreuses applications dans lesquelles le recours à un accumulateur électrique est exclu pour les raisons mentionnées plus haut. Si, pour diverses raisons, un accumulateur demeure nécessaire, ce dispositif permet d'améliorer sensiblement le bilan énergétique.

Suivant une caractéristique de l'invention, le circuit d'alimentation comprend la combinaison d'une diode série et d'un condensateur parallèle.

Suivant une caractéristique de l'invention, lesdits moyens de détection comprennent des moyens de détection des variations de la puissance électrique consommée par ladite antenne émettrice.

Suivant une autre caractéristique de l'invention, lesdits moyens de détection comprennent des moyens de détection des variations du déphasage courant/tension dans l'antenne émettrice.

D'autres caractéristiques et avantages de l'invention ressortiront de la description qui va suivre de divers modes de réalisation donnés uniquement à titre d'exemples et illustrés par les dessins annexés sur lesquels :

- la figure 1 est un schéma synoptique illustrant le procédé de transmission suivant l'invention ;
- la figure 2 est un schéma bloc d'un dispositif de transmission selon l'invention ;
- la figure 3 est un schéma électrique d'une antenne réceptrice associée à un premier type de circuit de modulation de la partie imaginaire de l'impédance du système esclave ;
- la figure 4 est un schéma électrique d'une antenne réceptrice associée à un second type de circuit de modulation de la partie imaginaire de l'impédance du système esclave ;
- la figure 5 est un schéma électrique d'une antenne réceptrice associée à un premier type de circuit de modulation de la partie réelle de l'impédance du système esclave ;
- la figure 6 est un schéma électrique d'une antenne réceptrice associée à un second type de circuit de modulation de la partie réelle de l'impédance du système esclave ;
- la figure 7 est un schéma électrique illustrant un mode préféré de réalisation du second type de circuit de modulation de la figure 6 ;
- la figure 8 est un schéma électrique d'un premier type de circuit associé au système maître du dispositif pour la détection des données transmises depuis le système esclave ;
- la figure 9 est un schéma électrique illustrant un mode particulier de réalisation du premier type de circuit de détection de la figure 8 ;

- la figure 10 est un schéma électrique d'un second type de circuit associé au système maître pour la détection des données transmises depuis le système esclave ;
- la figure 11 est un schéma électrique illustrant un mode particulier de réalisation du second type de circuit de détection de la figure 10 ; et
- la figure 12 est un chronogramme illustrant des signaux générés dans un dispositif de transmission équipé des circuits des figures 7 et 11.

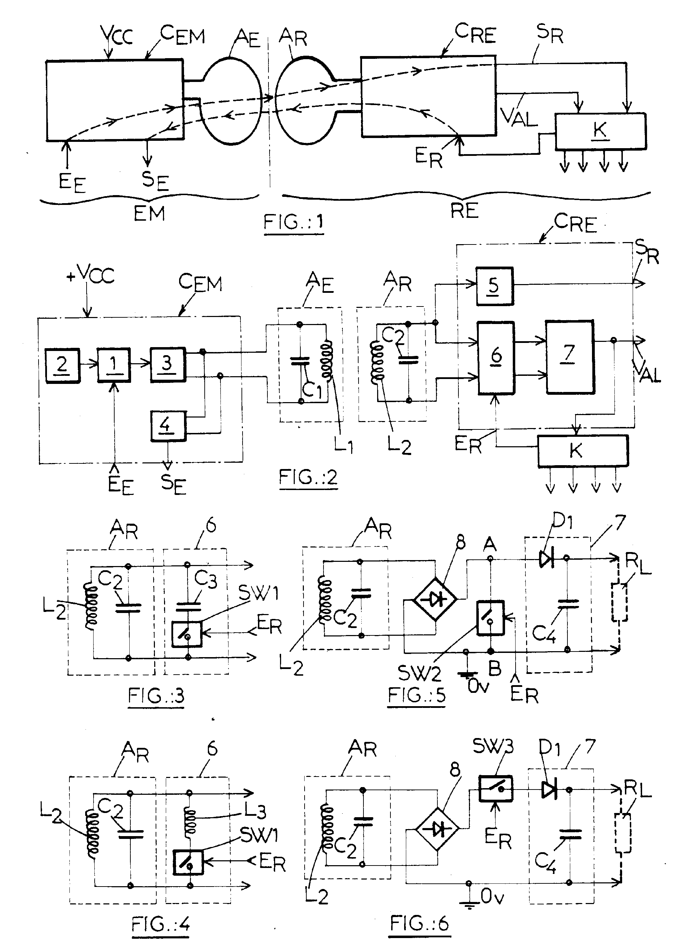

En se référant à la figure 1, le dispositif de transmission suivant l'invention comprend un système maître EM comportant une antenne émettrice $A_E$ et des circuits électriques d'émission et de traitement $C_{EM}$ connectés à une source d'alimentation électrique (non représentée) qui leur applique une tension $V_{cc}$. Ce système maître EM présente une entrée de données $E_E$ et une sortie de données $S_E$.

Un système esclave RE, dépourvu de toute connexion avec le système maître EM, comprend une antenne réceptrice $A_R$ et des circuits électriques de réception, d'alimentation et de modulation $C_{RE}$ délivrant sur une sortie $S_R$ les données reçues du système maître EM et sur une sortie $V_{AL}$ une tension continue d'alimentation. Le système esclave RE comprend également un bloc K symbolisant des circuits électriques tels que, par exemple, des circuits de traitement des données délivrées à la sortie $S_R$, des actionneurs, des circuits d'alimentation d'électrodes ou autres charges, des capteurs recueillant des données qui sont appliquées à une entrée $E_R$ du système esclave RE afin d'être transmises vers le système maître EM dans des conditions qui seront décrites ci-après, etc...

Les deux antennes $A_E$ et $A_R$ sont des circuits de type LC accordés sur une même fréquence porteuse f et couplés de sorte que toute modification du comportement électrique de l'un des systèmes se répercute sur l'autre système. En fonctionnement, le système maître EM émet sur la fréquence f qui est modulée, par exemple en tout ou rien, par les données appliquées à l'entrée $E_E$. Le signal capté par l'antenne réceptrice $A_R$ en provenance de l'antenne émettrice $A_E$ véhicule à la fois ces données et de l'énergie nécessaire à l'alimentation du système esclave RE. Les circuits électriques $C_{RE}$ comprennent des circuits conventionnels de réception et d'alimentation permettant, d'une part, d'extraire et de mettre en forme les données transmises par le système maître EM, dont le cheminement est symbolisé par la ligne en traits interrompus s'étendant entre l'entrée $E_E$ et la sortie $S_R$ et, d'autre part, de convertir l'énergie reçue par l'antenne $A_R$ en une tension d'alimentation continue $V_{AL}$ qui alimente l'ensemble des circuits côté système esclave RE.

Les données appliquées à l'entrée $E_R$ par les circuits du bloc K sont transmises au système maître EM grâce à une modulation de l'impédance du système esclave RE qui a lieu pendant que le système maître EM émet. Cette transmission de données est symbolisée par la ligne en traits interrompus qui s'étend entre l'entrée $E_R$ du système esclave RE et la sortie $S_E$ du système maître EM. Le processus qui préside à cette transmission est le suivant : comme il y a variation de couplage entre le système maître et le système esclave, l'impédance $Z_E$ de l'antenne d'émission $A_E$, vue par le système maître EM, est de la forme :

$$Z_E = Z_O + \frac{M^2 \omega^2}{Z_R}$$

où,

- $Z_O$ représente l'impédance de l'antenne d'émission $A_E$ dans le cas d'un système esclave placé infiniment loin,
- $Z_R$ représente l'impédance du système esclave RE (antenne réceptrice $A_R$ et circuits associés);
- M représente le coefficient de couplage entre l'antenne émettrice et l'antenne réceptrice, coefficient qui dépend des caractéristiques géométriques des antennes (diamètre, éloignement) et de la qualité de couplage de ces antennes, et
- $\omega$ représente la pulsation de la fréquence émise par le système maître EM.

Par conséquent, en modulant l'impédance $Z_R$ on induit des variations de l'impédance $Z_E$ qui peuvent être détectées au niveau de le système maître EM et permettent de reconstituer les données qui ont été appliquées à l'entrée $E_R$ du système esclave RE.

Le procédé de transmission qui vient d'être décrit permet ainsi une transmission bidirectionnelle d'informations entre le système maître et le système esclave sur une seule fréquence, et ceci au moyen d'une seule antenne aussi bien côté système maître que côté système esclave. Il en résulte par conséquent une économie de place tant côté système maître, que système esclave, et une économie de puissance au niveau du système esclave car, comme cela ressortira de la description qui va suivre, l'énergie nécessaire pour moduler l'impédance du système esclave est extrêmement faible. Ce point est très important puisque le système esclave ne tire de l'énergie que du signal reçu du système maître et que cette énergie doit être utilisée en priorité pour alimenter un certain nombre de charges électriques telles que des actionneurs, des capteurs, différents circuits électroniques, etc... qui sont associés au système esclave.

En se reportant à la figure 2, les circuits de réception et de traitement $C_{EM}$ comprennent un modulateur 1 qui module le signal à fréquence constante délivré par un oscillateur pilote 2, en fonction d'un signal de modulation qui lui est appliquée à l'entrée $E_E$. La modulation est de préférence du type tout ou rien, c'est à dire que le signal de l'oscillateur pilote 2 est appliqué par le modulateur 1 à l'entrée d'un amplificateur de puissance 3 (puissance nécessaire à l'alimentation du système esclave) lorsque le signal de modulation se trouve dans un état haut et qu'aucun signal n'est appliqué à l'entrée de l'amplificateur de puissance 3 lorsque le signal de modulation se trouve à l'état bas, ou inversement. L'antenne d'émission $A_E$ est de type classique à inductance L1 et capacité $C_1$.

Les circuits $C_{EM}$ sont complétés par un circuit 4 de détection ou mesure des variations d'une caractéristique électrique induites dans le système maître EM par la modulation de l'impédance du système esclave. La caractéristique dont les variations sont détectées peut être une grandeur représentative du module ou de l'argument de l'impédance de l'antenne émettrice $A_E$.

Côté système esclave, l'antenne $A_R$ est également une antenne accordée du type à inductance $L_2$ et condensateur $C_2$. Les circuits $C_{RE}$ comprennent des circuits électroniques de réception conventionnels 5 qui démodulent et mettent en forme le signal reçu par l'antenne réceptrice $A_R$ et délivrent sur la sortie $S_R$ les données transmises par le système maître EM.

L'antenne $A_R$ est également couplée à un circuit modulateur d'impédance 6 qui reçoit les données à transmettre vers le système maître EM sur son entrée $E_R$. Les circuits $C_{RE}$ sont complétés par un circuit d'alimentation conventionnel 7 qui convertit l'énergie reçue par l'antenne réceptrice $A_R$ en une tension continue d'alimentation $V_{AL}$ qui sert à alimenter les différents circuits ou charges K connectés au système esclave, en particulier des capteurs appliquant des données à l'entrée $E_R$ du circuit de modulation 6.

L'impédance $Z_R$ du système esclave RE rapportée à l'antenne d'émission $A_E$ est de la forme R + j X où R représente la partie réelle de cette impédance et X sa partie imaginaire. La partie réelle R est essentiellement constituée par les pertes dues au fait que le système esclave consomme de l'énergie et présente une certaine résistance équivalente ramenée au système maître. La partie imaginaire X est pratiquement constante pour une fréquence porteuse et un accord d'antenne donnés. Pour moduler l'impédance $Z_R$, il est donc possible de faire varier sa partie réelle et / ou sa partie imaginaire.

Le schéma de la figure 3 représente un premier circuit associé à l'antenne réceptrice $A_R$ pour faire varier la partie imaginaire X de l'impédance $Z_R$. Ce premier circuit comprend, en parallèle avec l'inductance $L_2$ et le condensateur $C_2$, l'association en série d'un condensateur $C_3$ et d'un commutateur électronique SW1 piloté par les données appliquées à l'entrée $E_R$ du système esclave.

Ainsi, lorsque le commutateur $SW_1$ est ouvert, l'impédance du système esclave RE n'est pas affectée, lorsque ce commutateur est fermé le condensateur $C_3$ se trouve connecté en parallèle avec le condensateur $C_2$ et la partie imaginaire X de l'impédance $Z_R$ se trouve modifiée. Les données appliquées à l'entrée de commande $E_R$ du commutateur SW1 sont constituées par un signal à deux états dont l'un impose la fermeture du commutateur et l'autre son ouverture.

Le circuit de modulation 6 de la figure 4 diffère de celui de la figure 3 uniquement en ce que le condensateur $C_3$ est remplacé par une inductance $L_3$. Comme dans le cas de la figure 3, la mise en circuit ou hors circuit de l'inductance $L_3$ a pour effet de modifier la partie imaginaire de l'impédance $Z_R$.

Les circuits des figures 3 et 4 offrent la possibilité de faire varier fortement la partie imaginaire X de l'impédance $Z_R$ et de faciliter ainsi la mesure des variations de l'impédance $Z_E$ de l'antenne d'émission. En contrepartie, le commutateur SW1, par exemple un transistor, doit être en mesure de commuter un signal alternatif, présenter une capacité parasite très faible et être capable de supporter des tensions élevées de l'ordre de, par exemple 100 volts, car il est fréquemment nécessaire de disposer de telles tensions élevées pour assurer le fonctionnement de charges ou circuits électroniques associés au système esclave.

Une autre possibilité illustrée par les figures 5 et 6 consiste à faire varier la partie réelle R de l'impédance $Z_R$.

A la figure 5, l'antenne $A_R$ est connectée au circuit d'alimentation 7 par l'intermédiaire d'un pont redresseur classique 8. Le circuit 7 alimente l'ensemble des circuits électroniques, capteurs et autres organes consommateurs d'énergie électrique (bloc K) symbolisés par une charge $R_L$ sur la figure. Ce circuit 7 est constitué classiquement d'une diode $D_1$ disposée en série dans l'une des branches de sortie du pont redresseur 8 et d'un condensateur de stockage $C_4$ connecté, entre les branches de sortie du pont, en aval de la diode $D_1$. Un commutateur électronique SW2 est également connecté entre les branches de sortie du pont redresseur 8, entre celui-ci et la diode $D_1$. L'électrode de commande du commutateur SW2 constitue l'entrée $E_R$ à laquelle sont appliquées les données à transmettre au système maître EM.

Dans ce montage, la modulation effectuée au moyen du commutateur SW2 a pour effet d'agir sur la consommation continue du système esclave. Le circuit de modulation a l'avantage d'être simple car il ne commute que des tensions continues présentant une référence par rapport à la masse (O volt). En fonctionnement, lorsque le commutateur SW2 est fermé, l'impédance vue entre ses bornes A et B est faible. La diode $D_1$ empêche que le condensateur $C_4$ ne se décharge complètement à travers le commutateur SW2. Lorsque ce dernier est ouvert, le condensateur $C_4$, qui s'était légèrement déchargé dans la charge $R_L$, se recharge en présentant une impédance faible. Par conséquent, la partie réelle R de l'impédance $Z_R$ ne varie que faiblement, surtout en régime transitoire, et la détection des variations induites côté système maître est relativement délicate à assurer.

De ce point de vue, le circuit de la figure 6 donne davantage satisfaction. Ce circuit diffère de celui de la figure 5 en ce que le commutateur SW3 est connecté, non pas entre les branches de sortie du pont redresseur 8, mais en série entre l'une des branches de sortie du pont redresseur 8 et l'anode de la diode $D_1$, l'autre branche du pont diviseur étant celle qui est connectée à la masse. Comme dans le cas des figures 3 à 5, l'électrode de commande du commutateur SW3 constitue l'entrée de données $E_R$ du système esclave.

En fonctionnement, lorsque le commutateur SW3 est ouvert, la partie réelle R de l'impédance $Z_R$ représente les pertes dans l'antenne $A_R$ et R est alors élevé. Lorsque le commutateur SW3 est fermé la partie réelle R est représentée par la mise en parallèle des pertes ohmiques dans l'antenne avec la charge $R_L$ représentant l'ensemble des éléments consommateurs d'énergie. La partie réelle R est alors petite, elle est encore diminuée pendant les périodes transitoires pendant lesquelles le condensateur $C_4$ doit se recharger.

Le circuit de la figure 6 permet donc d'induire des variations importantes de la partie réelle R de l'impédance $Z_R$ et de détecter ainsi aisément les variations induites sur l'impédance $Z_E$ de l'antenne émettrice $A_E$. Par contre, le commutateur électronique SW3 se trouve référencé par rapport à-la tension de sortie du redresseur, ce qui conduit à utiliser un transistor PNP ou PMOS plus difficile à piloter que dans le cas de la figure 5.

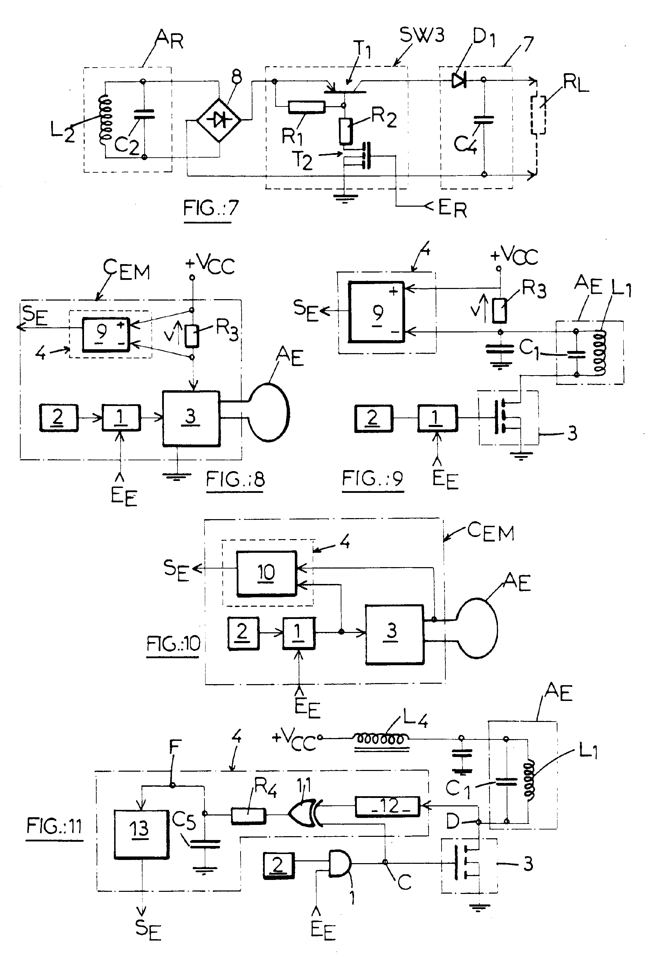

La figure 7 montre une forme particulière de réalisation du commutateur SW3 de la figure 6. Ce commutateur comprend un transistor PNP $T_1$ dont l'émetteur est connecté à la branche de sortie du pont redresseur 8 délivrant la tension d'alimentation et dont le collecteur est connecté à l'anode de la diode $D_1$. L'émetteur du transistor $T_1$ est connecté à sa base par une résistance $R_1$. La base du transistor $T_1$ est connecté à l'autre branche de sortie (masse) du pont redresseur 8 par l'intermédiaire d'une résistance $R_2$ et du trajet drain-source d'un transistor $T_2$ de type MOS-FET. La grille du transistor $T_2$ constitue l'entrée $E_R$ à laquelle sont appliquées les données à transmettre du système esclave au système maître.

En fonctionnement, lorsque le signal appliqué à la grille du transistor $T_2$ par l'entrée $E_R$ est au niveau bas, le transistor $T_2$ est bloqué il en va de même du transistor $T_1$ dont la base est au même potentiel que l'émetteur. Le commutateur SW3 est donc ouvert. Quand un niveau haut est appliqué à l'entrée $E_R$, le transistor $T_2$ est rendu conducteur et débloque le transistor $T_1$ dont la base se trouve polarisée négativement par rapport à son émetteur. Le commutateur SW3 est alors en position fermée.

Le schéma bloc de la figure 8 illustre un premier type de circuit 4 de détection des variations de l'impédance $Z_E$ induites par la modulation de l'impédance $Z_R$. Le fonctionnement de ce circuit 4 de détection est basé sur le fait que, lorsqu'on modifie l'impédance $Z_R$, on modifie son module $/Z_R/ = \sqrt{R^2 + X^2}$, et on désaccorde l'antenne $A_R$. Par conséquent, on modifie la consommation électrique continue du circuit d'émission du système maître EM car l'énergie émise par l'antenne d'émission $A_E$ est partiellement réfléchie par l'antenne de réception $A_R$. Dans ce mode de détection, l'amplificateur 3 qui pilote l'antenne d'émission $A_E$ doit être de type classe B ou classe C afin d'avoir une consommation électrique sur l'alimentation continue $+V_{cc}$ qui varie avec l'impédance $Z_E$.

Le circuit de détection 4 est constitué par un amplificateur différentiel 9 qui est connecté aux bornes d'une résistance $R_3$ traversée par le courant $I_{cc}$ d'alimentation de l'amplificateur de puissance 3. La tension mesurée aux bornes de la résistance $R_3$ par l'amplificateur différentiel 9 est représentative de ce courant $I_{cc}$. Le courant $I_{cc}$ varie lui-même avec le module de l'impédance $Z_R$ et, par conséquent, l'amplificateur différentiel 9 délivre à la sortie $S_E$ un signal qui est l'image des données appliquées à l'entrée $E_R$ du système maître. Si nécessaire, le signal délivré par l'amplificateur différentiel 9 peut être mise en forme par des circuits conventionnels (non représentés).

La figure 9 illustre un mode de réalisation particulier du circuit de la figure 8, où l'amplificateur 3 est constitué par un transistor MOSFET dont la grille est commandée par le modulateur 1 et le trajet drain-source est connecté entre l'une des bornes de l'antenne $A_E$ et la masse, l'autre borne de l'antenne étant connectée au pôle positif $+V_{cc}$ de la source d'alimentation par l'intermédiaire de la résistance $R_3$. Dans ce mode de détection des variations de l'impédance $Z_E$, l'amplificateur 3 doit avoir la plus faible consommation possible à vide pour présenter un bon rapport signal/bruit.

Un autre mode de détection des variations d'impédance induites au niveau de l'antenne d'émission $Z_E$ consiste à détecter les variations du déphasage courant/tension dans l'antenne émettrice $A_E$. En effet, lorsqu'on fait varier la partie réelle R ou la partie imaginaire X de l'impédance $Z_R$, son argument $\Phi = \mathrm{arctg}\, \dfrac{X}{R}$ varie.

Le schéma bloc de la figure 10 illustre un moyen de mise en oeuvre de ce mode de détection. Sur cette figure, le circuit de détection 4 est constitué par un détecteur de phase ou phasemètre 10 dont les deux entrées sont connectées respectivement à l'entrée et à la sortie de l'amplificateur de puissance 3. En effet, pour un dispositif amplificateur, il existe une relation entre la tension d'entrée et la tension de sortie et cette relation est normalement dépendante de la nature de la charge. Par conséquent, le déphasage entrée/sortie de l'amplificateur 3 traduit le déphasage courant/tension dans l'antenne $A_E$. Le détecteur de phase 10 produit à sa sortie $S_E$ un signal représentatif de ce déphasage et, par conséquent, du signal appliqué à l'entrée $E_R$ du système esclave RE.

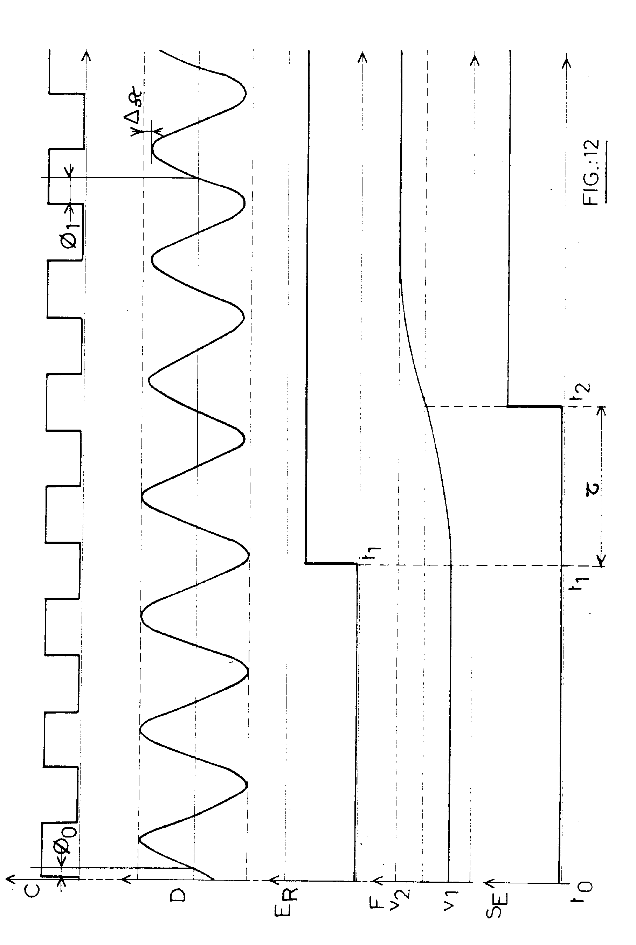

La figure 11 illustre un mode particulier de réalisation des circuits de la figure 10. Le modulateur 1 est constitué par une porte ET qui reçoit à l'une de ses entrées la porteuse délivrée par l'oscillateur pilote 2 et à son autre entrée $E_E$ le signal à deux états à transmettre vers le système esclave. Quand ce signal est au niveau haut, la porte ET applique la porteuse à l'amplificateur de puissance 3, quand il est au niveau bas la porte ET est bloquée et la porteuse n'est pas transmise. L'amplificateur de puissance 3 est constitué par un transistor MOSFET dont la grille C est connectée à la sortie de la porte ET, dont le drain D est connecté à l'une des bornes de l'antenne d'émission $A_E$ et dont la source est connectée à la masse. L'autre borne de l'antenne $A_E$ est connectée au pôle positif $+V_{cc}$ de la source d'alimentation par l'intermédiaire d'une self choc $L_4$.

Le détecteur de phase 10 comprend une porte OU EXCLUSIF 11 dont l'une des entrées est connectée à la grille C du transistor 3 et dont l'autre entrée est connectée par l'intermédiaire d'un écrêteur 12, au drain D de ce transistor. En effet, la détection du déphasage se fait entre grille et drain du transistor car le gain en tension d'un tel montage (source commune) vaut -gmZ.

La sortie de la porte 11 attaque l'entrée d'un circuit RC comprenant une résistance série $R_4$ et un condensateur en parallèle $C_5$. La sortie E du circuit RC est connectée à l'entrée d'un circuit de mise en forme 13, dont la sortie constitue la sortie de données $S_E$ du système maître EM.

Le fonctionnement du circuit de la figure 11 est illustré par le chronogramme de la figure 12 sur lequel :
- le signal C est celui appliqué à la grille du transistor 3 de la figure 11 (point C) ;
- le signal D est celui présent sur le drain du transistor 3 de la figure 11 (point D) ;
- le signal E est celui présent à la sortie du circuit $R_4$-$C_5$ de la figure 11 (point E) ;
- le signal $S_E$ est celui présent à la sortie du circuit de mise en forme 13 de la figure 11 ;
- le signal $E_R$ est celui appliqué à la grille du transistor $T_2$ de la figure 7 ;

Entre les instants $t_0$ et $t_1$, l'entrée $E_R$ (grille F du transistor) $T_2$ est au niveau bas et il n'y a pas de modification de l'impédance $Z_R$. Par conséquent, le déphasage (décalage relatif des passages par zéro) entre le signal en créneaux C et le signal alternatif D est limité à une faible valeur constante $\Phi_0$ et le signal au point F reste au niveau bas grâce à la constante de

temps du circuit $R_4$ $C_5$ qui lisse l'impulsion de courte durée (non représentée) engendrée à la sortie de la porte OU EXCLUSIF 11 en raison du déphasage constant $\Phi_0$. Lorsque, à l'instant $t_1$, l'entrée $E_R$ passe au niveau haut, le déphasage entre les signaux présents aux points C et D passe progressivement de $\Phi_0$ à $\Phi_1$ et le signal F de sortie du circuit $R_4$ $C_5$ passe progressivement d'une tension $V_1$ à une tension $V_2$. Lorsqu'à l'instant $T_2$, le signal F atteint le seuil $V_2$ du circuit de mise en forme 13, la sortie $S_E$ de ce dernier passe au niveau haut.

L'intervalle $t_1$-$t_2$ représente un temps de réponse $\tau$ qui dépend de la fréquence f de la porteuse et du facteur de qualité Q des antennes émettrice et réceptrice et qui limite la cadence maximale à laquelle des données peuvent être transmises du système esclave au système maître. En pratique, la bande passante utile s'étend de zéro au tiers de la fréquence porteuse environ.

Lorsque le signal $E_R$ repasse à l'état bas, le déphasage repasse progressivement de $\Phi_1$ à $\Phi_0$ et $S_E$ repasse à l'état bas au bout du temps de réponse $\tau$.

En modulant l'impédance du système esclave RE pendant les phases d'émission d'un signal par le système maître, on peut ainsi récupérer de manière très simple des données au niveau du système maître EM. Différents modes de codage des informations transmises du système maître EM au système esclave RE et, en sens inverse, du système esclave au système maître, peuvent être utilisées. C'est ainsi, par exemple, que les informations peuvent être codées en impulsions à largeur variable pour la transmission de données analogiques, ou en impulsions à largeur constante pour la transmission de données numériques. Il n'entre pas dans le cadre de la présente invention de décrire les différents modes de codage possibles, qui sont bien connus des spécialistes des techniques de transmission d'informations.

Le dispositif de transmission selon l'invention peut être avantageusement appliqué à des dispositifs électroniques actifs implantables dans le corps humain ou animal. Le système esclave RE constitue alors le dispositif électronique implanté qui peut être interrogé et/ou piloté à la demande, par exemple pour commander la durée et l'intensité du courant fourni à une ou plusieurs électrodes de stimulation. Les informations transmises au système maître par le dispositif électronique implanté peuvent consister, par exemple, en des mesures d'impédance entre électrodes au moyen de circuits classiques de mesure d'impédance qu'il n'y a pas lieu de décrire ici en détail. Ces informations peuvent également provenir de différents capteurs implantés et être transmises en série au système maître. En variante, les capteurs peuvent être interrogés sélectivement.

Les différents modes de modulation de l'impédance $Z_R$ n'ont pas la même efficacité suivant les valeurs de f et de Q. C'est ainsi, par exemple, que le mode de modulation illustré par la figure 3 est relativement peu efficace à une fréquence f élevée car la variation de l'impédance $Z_R$ introduite par la connexion du condensateur $C_3$ en parallèle avec le condensateur $C_2$ est faible. Ce mode de modulation est par contre efficace à fréquence f faible, mais introduit en contrepartie une forte désadaptation de l'antenne $A_R$. La fréquence f de la porteuse sera généralement comprise entre 100 KHz et 100 MHz, bien que le dispositif suivant l'invention puisse fonctionner à des fréquences situées en dehors de cette fourchette.

Sachant que le débit d'informations du système esclave vers le système maître est aussi une fonction de la fréquence f de la porteuse et du facteur de qualité Q des antennes, le choix d'un mode de modulation pour une application donnée est affaire de compromis entre les débits d'informations aller et retour et l'efficacité du transfert d'énergie vers le système esclave et peut être effectué par un spécialiste de la radioélectricité se contentant d'appliquer ses connaissances usuelles.

Il va de soi que les modes de réalisation décrits ne sont que des exemples et l'on pourrait les modifier par substitution d'équivalents techniques sans sortir du cadre de l'invention.

## Revendications

1. Dispositif de transmission bidirectionnelle d'informations entre un système maître et un système esclave comportant respectivement une antenne émettrice unique et une antenne réceptrice unique accordées sur une même fréquence,
   - ledit système maître comprenant :
     * des moyens pour transmettre des premières informations au système esclave par modulation d'un signal électromagnétique émis à ladite fréquence,
     * des moyens pour détecter, pendant ladite émission dudit signal électromagnétique modulé, des variations de caractéristiques électriques induites par une modulation de l'impédance du système esclave fonction de secondes informations à transmettre du système esclave au système maître, et
     * des moyens pour reconstituer lesdites secondes informations en fonction desdites variations de caractéristiques électriques détectées,
   - ledit système esclave comprenant un circuit résonant de réception accordé sur ladite fréquence et comprenant ladite antenne réceptrice,
     
           caractérisé en ce que ledit système esclave (RE) comprend en outre :

* un circuit redresseur (8) connecté aux bornes dudit circuit résonant de réception ($L_2$, $C_2$),
* un circuit d'alimentation (7) générateur de tension continue connecté audit circuit redresseur (8) pour alimenter des circuits et/ou charges dudit système esclave, et
* un circuit de commutation ($SW_3$) connecté en série entre ledit circuit redresseur (8) et ledit circuit d'alimentation (7) pour faire varier la partie réelle de l'impédance dudit système esclave (RE) entre une valeur élevée et une valeur faible suivant que ledit commutateur ($SW_3$) est ouvert ou fermé sous la commande d'un signal représentatif desdites secondes informations à transmettre au système maître (EM).

2. Dispositif selon la revendication 1, caractérisé en ce que ledit circuit de commutation ($SW_3$) comprend un premier transistor ($T_1$) dont la jonction émetteur-collecteur est connectée en série entre ledit circuit redresseur (8) et ledit circuit d'alimentation (7), une première résistance de polarisation ($R_1$) connectée entre la base dudit transistor ($T_1$) et ledit circuit redresseur (8), et une seconde résistance ($R_2$) connectée entre ladite base et la masse.

3. Dispositif selon la revendication 2, caractérisé en ce que ledit circuit de commutation ($SW_3$) comprend un second transistor ($T_2$) dont le trajet drain-source est connecté en série entre ladite seconde résistance ($R_2$) et la masse et dont la grille reçoit ledit signal ($E_R$) représentatif desdites secondes informations.

4. Dispositif selon l'une quelconque des revendications 1 à 3, caractérisé en ce que ledit circuit d'alimentation (7) comprend la combinaison d'une diode série ($D_1$) et d'un condensateur parallèle ($C_4$).

5. Dispositif selon l'une quelconque des revendications 1 à 4, caractérisé en ce que lesdites moyens de détection (4) comprennent des moyens (9, $R_3$) de détection des variations de la puissance électrique consommée par ladite antenne émettrice ($A_E$).

6. Dispositif selon l'une quelconque des revendications 1 à 4, caractérisé en ce que lesdits moyens de détection (4) comprennent des moyens (11-13, $R_4$, $C_5$) de détection des variations du déphasage courant/tension dans l'antenne émettrice ($A_E$).

7. Dispositif selon l'une quelconque des revendications 1 à 6, caractérisé en ce que ledit système esclave (R$_E$) constitue un dispositif électronique actif implantable dans le corps humain ou animal.

**Patentansprüche**

1. Vorrichtung zur bidirektionalen Übertragung von Informationen zwischen einem Hauptsystem und einem Untersystem, die jeweils eine einzige Sendeantenne und eine einzige Empfangsantenne aufweisen, welche auf die gleiche Frequenz abgestimmt sind,
   - wobei das Hauptsystem umfaßt:
     * Einrichtungen zur Übertragung von ersten Informationen auf das Untersystem durch Modulation eines mit der vorstehenden Frequenz abgegebenen elektromagnetischen Signales,
     * Einrichtungen zum Detektieren von Veränderungen der elektrischen Eigenschaften, die durch eine Modulation der Impedanz des Untersystems in Abhängigkeit von zweiten vom Untersystem auf das Hauptsystem zu übertragenen Informationen induziert sind, während des Sendens des modulierten elektromagnetischen Signales und
     * Einrichtungen zum Wiederherstellen der zweiten Informationen in Abhängigkeit von den detektierten Änderungen der elektrischen Eigenschaften,
   - wobei das Untersystem einen Empfangsschwingkreis umfaßt, der auf die genannte Frequenz abgestimmt ist und die Empfangsantenne aufweist, dadurch gekennzeichnet, daß das Untersystem (RE) u. a. aufweist:
     * einen Gleichrichterkreis (8), der an die Klemmen des Empfangsschwingkreises (L$_2$, C$_2$) angeschlossen ist,
     * einen Speisestromkreis (7), der eine Gleichspannung erzeugt und an den Gleichrichterkreis (8) angeschlossen ist, um die Kreise und/oder Lasten des Untersystems zu speisen, und
     * einen Schaltkreis (SW$_3$), der in Reihe zwischen den Gleichrichterkreis (8) und den Speisestromkreis (7) geschaltet ist, um den Nutzteil der Impedanz des Untersystems (RE) zwischen einem hohen Wert und einem geringen Wert in Abhängigkeit davon, ob der Schalter (SW$_3$) offen oder geschlossen ist, unter dem Befehl eines auf das Hauptsystem (EM) zu übertragenden, für die zweiten Informationen repräsentativen Signales zu verändern.

2. Vorrichtung nach Anspruch 1, dadurch gekennzeichnet, daß der Schaltkreis (SW$_3$) einen ersten Transistor (T1), dessen Übergang Emitter-Kollektor in Reihe zwischen den Gleichrichterkreis (8) und den Speisestromkreis (7) geschaltet ist, und einen ersten Polarisationswiderstand (R$_1$), der zwischen die Basis des Transistors (T$_1$) und den Gleichrichterkreis (8) geschaltet ist, sowie einen zweiten Widerstand (R$_2$), der zwischen die Basis und Masse geschaltet ist, umfaßt.

3. Vorrichtung nach Anspruch 2, dadurch gekennzeichnet, daß der Schaltkreis (SW$_3$) einen zweiten Transistor (T$_2$) aufweist, dessen Weg Drain-Source in Reihe zwischen den zweiten Widerstand (R$_2$) und Masse geschaltet ist und dessen Gate das für die zweiten Informationen repräsentative Signal (E$_R$) empfängt.

4. Vorrichtung nach einem der Anprüche 1-3, dadurch gekennzeichnet, daß der Speisestromkreis (7) eine Kombination aus einer Reihendiode (D$_1$) und einem Parallelkondensator (C$_4$) umfaßt.

5. Vorrichtung nach einem der Ansprüche 1-4, dadurch gekennzeichnet, daß die Detektionseinrichtungen (4) Einrichtungen (9, R$_3$) zum Detektieren von Veränderungen der von der Sendeantenne (A$_E$) verbrauchten elektrischen Energie umfassen.

6. Vorrichtung nach einem der Ansprüche 1-4, dadurch gekennzeichnet, daß die Detektionseinrichtungen (4) Einrichtungen (11-13, R$_4$, C$_5$) zum Detektieren von Veränderungen der Phasenverschiebung Strom/Spannung in der Sendeantenne (A$_E$) umfassen.

7. Vorrichtung nach einem der Ansprüche 1-6, dadurch gekennzeichnet, daß das Untersystem (R$_E$) eine aktive elektronische Vorrichtung bildet, die in den menschlichen oder tierischen Körper implantierbar ist.

**Claims**

1. A device for the bidirectional transmission of data between a master system and a slave system comprising respectively a single transmission antenna and a single reception antenna which are tuned to a same frequency,
   - said master system comprising :
     * means for transmitting first data to said slave system by modulating an electromagnetic signal transmitted at said frequency ;

* means for detecting, during said transmission of said modulated electromagnetic signal, variations in electrical characteristics induced by a modulation of the impedance of said slave system as a function of second data to be transmitted from said slave system to said master system, and

* means for restoring said second data on the basis of said detected variations in electrical characteristics,

- said slave system comprising a reception resonant circuit tuned to said frequency and comprising said reception antenna, characterized in that said slave system (RE) further comprises:

- a rectifier circuit (8) connected across said reception resonant circuit ($L_2$, $C_2$),

- a power supply circuit (7) connected to said rectifier circuit (8) for powering circuits and/or loads of said slave system with a DC voltage, and

- a switching circuit (SW3) connected in series between said rectifier circuit (8) and said power supply circuit (7) in order to modulate the real part of the impedance of said slave system (RE) between a high value and a low value when said switching circuit (SW3) is driven in its open or close position by a control signal representative of said second data to be transmitted to the master system (EM).

2. Device according to claim 1, characterized in that said switching circuit (SW3) comprises a first transistor ($T_1$) having its emitter-collector path connected in series between said rectifier circuit (8) and said power supply circuit (7), a first polarization resistor ($R_1$) connected between the base of said transistor ($T_1$) and said rectifier circuit (8), and a second polarization resistor ($R_2$) connected between said base and ground.

3. Device according to claim 2, characterized in that said switching circuit (SW3) comprises a second transistor ($T_2$) having its drain-source path connected in series between said second resistor ($R_2$) and ground and receiving on its grid said signal ($E_R$) representative of said second data.

4. Device according to any one of claims 1 to 3, characterized in that said power supply circuit (7) includes the combination of a series diode ($D_1$) and a parallel capacitor ($C_4$).

5. Device according to any one of claims 1 to 4, characterized in that said detection means (4) include means (9, $R_3$) of detecting variations in the electrical power consumed by said transmission antenna ($A_E$).

6. Device according to any one of claims 1 to 4, characterized in that said detection means (4) include means (11-13, $R_4$, $C_5$) of detecting variations in the current/voltage phase lag in the transmission antenna ($A_E$).

7. Device according to any one of claims 1 to 6, characterized in that said slave system (RE) constitutes an active electronic device which can be implanted in a human or animal body.

FIG.:1

FIG.:2

FIG.:3

FIG.:4

FIG.:5

FIG.:6

11

FIG.:7

FIG.:8

FIG.:9

FIG.:10

FIG.:11

FIG.:12

EP 0 464 180 B1